# EUROPÄISCHE PATENTANMELDUNG

(11) **EP 4 748 434 A2**
(43) Veröffentlichungstag der Anmeldung: **27.05.2026**
(21) Anmeldenummer: 26162089.2
(22) Anmeldetag: 09.10.2014
(51) Int. Cl.: A61M 60/205

(54) **KOMPRIMIERBARER MOTOR, IMPLANTIERANORDNUNG SOWIE VERFAHREN ZUM POSITIONIEREN DES MOTORS**

(30) Priorität: 11.10.2013 EP 13188380
(62) Teilanmeldung aus: 21161641.2
(71) Anmelder: ECP Entwicklungsgesellschaft mbH, 52074 Aachen (DE)
(72) Erfinder: SCHUMACHER, Jörg, 52074 Aachen (DE)
(74) Vertreter: Pfenning, Meinig & Partner mbB

(57) **Zusammenfassung**

Die Erfindung bezieht sich auf einen Motor mit einem Stator (2, 2') und einem um eine Axialrichtung (4) antreibbaren Rotor (1, 1'), dadurch gekennzeichnet, dass von diesen wenigstens einer, insbesondere der Stator, der eine strombeaufschlagbare Wicklungsanordnung aufweist, radial komprimierbar und expandierbar ist.

## Beschreibung

Die Erfindung liegt auf dem Gebiet der Mechanik und Elektrotechnik und ist mit besonderem Vorteil im Bereich der Mikromechanik einsetzbar. Unter anderem sind Anwendungen in der Medizintechnik besonders vorteilhaft.

Beim Einbringen von Aggregaten in Kanalsysteme bzw. an besonders schwierig zugängliche Orte ergeben sich, wie auch beim Implantieren von Aggregaten in einen Patientenkörper, oft Probleme dadurch, dass derartige Aggregate durch möglichst enge Durchgangsöffnungen oder Kanäle an ihre Sollpositionen gebracht werden müssen, einmal an der Zielposition angelangt jedoch eine möglichst große Wirkung, unter anderem auch durch möglichst große Abmessungen, entfalten sollen.

In der Medizintechnik ist hierzu die Vorgehensweise bekannt, entsprechende Aggregate vor dem Einbringen in den Patientenkörper und zum Zielort zu komprimieren, diese im komprimierten Zustand einzuführen und danach zu expandieren. Dies ist bisher bereits bei expandierbaren Katheterpumpen zur Herzunterstützung und auch bei implantierbaren Stents angewendet worden. Im nichtmedizinischen Bereich können beispielsweise Inspektionswagen durch Rohre geschickt werden, die beim Erreichen größerer Hohlräume ebenfalls expandiert werden können oder bei denen geeignete Werkzeuge oder Sensoren ausgefahren werden können.

Im medizinischen Bereich werden antreibbare expandierbare Aggregate bisher, wenn sie implantiert sind, durch körperexterne Motoren mittels flexibler Wellen angetrieben. Bei Blutpumpen zur Herzunterstützung ist beispielsweise vorgesehen, dass ein Pumpenrotor mittels einer flexiblen Welle, die durch einen Hohlkatheter innerhalb von Blutgefäßen verläuft, mit einem körperexternen Motor durch eine Schleuse verbunden ist. An derartige Übertragungssysteme, beispielsweise in Form von flexiblen Wellen, werden teilweise sehr hohe Anforderungen gestellt, da diese hohe Drehzahlen über längere Zeit ohne nennenswerten Abrieb und unter meist nicht optimalen Schmierungsbedingungen übertragen müssen. Es wäre daher von Vorteil, wenn auf derartige Übertragungssysteme verzichtet werden könnte.

Vor dem Hintergrund des Standes der Technik liegt daher der vorliegenden Erfindung die Aufgabe zugrunde, einen Motor derart zu gestalten, dass er auch durch enge Kanäle transportierbar ist.

Die Aufgabe wird den Merkmalen der Erfindung gemäß Patentanspruch 1 gelöst. Der Patentanspruch 1 bezieht sich auf einen erfindungsgemäßen Motor, und die von diesem abhängigen Unteransprüche 2 bis 11 bezeichnen vorteilhafte Ausgestaltungen der Erfindung. Der Patentanspruch 12 bezieht sich auf eine Implantieranordnung und die Patentansprüche 13 und 14 auf ein Verfahren zum Positionieren eines erfindungsgemäßen Motors.

Der erfindungsgemäße Motor weist einen Stator und einen um eine Axialrichtung antreibbaren Rotor auf, wobei zur Lösung der Aufgabe von diesen wenigstens einer, insbesondere der Stator, welcher eine strombeaufschlagbare Wicklungsanordnung aufweist, radial komprimierbar und expandierbar ist.

Unter der radialen Komprimierbarkeit soll im Rahmen der vorliegenden Anmeldung verstanden werden, dass der Durchmesser des betrachteten Elements, bezogen auf die Rotationsachse des Motors, zumindest teilweise verringerbar ist. Dies kann alle Möglichkeiten einer gleichmäßigen Durchmesserverringerung umfassen, bei der ein zylindrischer Körper lediglich seinen Durchmesser verändert, ohne ansonsten seine Form zu ändern. Es können unter der radialen Komprimierbarkeit jedoch auch Durchmesserverringerungen des Stators und/oder Rotors in nur einer Achse verstanden werden, die beispielsweise durch Flachdrücken des Elements oder, falls das Element (Stator und/oder Rotor) aus verschiedenen Kreisscheiben zusammengesetzt ist, durch Kippen der Kreisscheiben gegenüber der Rotationsachse erzeugt werden. In dem genannten Fall wird der Durchmesser in einer ersten Richtung senkrecht zur Rotationsachse verringert, während er in der Richtung senkrecht zur ersten Richtung konstant bleibt.

Derartige komprimierbare Statoren mit einer strombeaufschlagbaren Wicklungsanordnung sind aus dem Stand der Technik nicht bekannt. Es kann erfindungsgemäß insbesondere die Wicklungsanrdnung selbst radial komprimierbar sein. Eine derartige Wicklungsanordnung kann vor dem Einbringen in einen Kanal komprimiert werden, wodurch besonders dann, wenn der Stator den Außendurchmesser des Motors bestimmt, der Gesamtdurchmesser des Motors reduziert wird. Beispielsweise kann vorgesehen sein, dass der Stator den Rotor im Betriebszustand koaxial umgibt. In diesem Fall ist eine radiale Komprimierung des Stators gleichbedeutend mit einer radialen Komprimierung des Motors. Der Stator kann noch von einem Gehäuse umgeben sein, das beispielsweise elastisch ausgebildet sein kann und dann mit dem Stator komprimierbar und expandierbar ist. Das Gehäuse kann beispielsweise im Wesentlichen aus einer elastischen Folie bestehen, die über den Stator gespannt ist. Es kann jedoch auch vorgesehen sein, dass der Motor ohne ein Gehäuse auskommt.

Wenn der Stator den Rotor koaxial umgibt, ist den Komprimierungsmöglichkeiten des Stators durch den innenliegenden Rotor eine enge Grenze gesetzt. Eine weitergehende Komprimierung des Stators ist beispielsweise vorteilhaft möglich, wenn Rotor und Stator gegeneinander in Axialrichtung verschiebbar sind zwischen einer ersten Position, in der der Stator radial komprimierbar ist, und einer zweiten Position, in der der Stator radial expandiert ist. In diesem Fall kann zur Komprimierung des Stators der Rotor zunächst aus diesem axial herausgeschoben werden, und darauf kann der Stator komprimiert werden, beispielsweise bis auf den Außendurchmesser des Rotors. Danach können Stator und Rotor axial hintereinander durch einen Kanal zur Sollposition geschoben werden. Ist der Rotor für sich nicht komprimierbar, so ist eine weitergehende Komprimierung des Stators unter das Außenmaß des Rotors in einigen Ausführungsbeispielen nicht vorgesehen bzw. nicht möglich.

Sind Stator und Rotor an der Sollposition angelangt, so kann der Stator wieder expandiert werden oder sich selbsttätig expandieren, und der Rotor kann in axialer Richtung in den Stator gezogen werden.

Es kann auch die radiale Expansion des Stators dadurch geschehen, dass der Rotor in den Stator verschoben bzw. gezogen wird und diesen bei der Verschiebungsbewegung radial expandiert. Zu diesem Zweck kann der Rotor zumindest abschnittsweise konisch zulaufend ausgebildet sein.

Eine weitere vorteilhafte Variante der Erfindung sieht vor, dass der Rotor in Radialrichtung komprimierbar ist. Ist auch der Rotor in Radialrichtung komprimierbar, so kann in einer Variante der Rotor im Stator verbleiben und beide können gemeinsam radial komprimiert werden, andererseits ist es auch denkbar, den Rotor aus dem Stator heraus zu verschieben und beide unabhängig voneinander radial zu komprimieren.

Beispielsweise kann dazu vorgesehen sein, dass der Rotor eine Mehrzahl von Magneten aufweist, die als Magnetelemente bezeichnet werden können und die insbesondere in Axialrichtung gegeneinander reversibel bewegbar sind. Der Rotor kann beispielsweise Permanentmagnete oder Elektromagnete mit einem ferromagnetischen Kern, die jeweils als Magnetelemente bezeichnet werden sollen, aufweisen. Solche Magneten können jeder für sich derart in Magnetsegmente geteilt sein, dass einzelne Segmente eines Magneten gegeneinander verschiebbar sind, um den Durchmesser des Rotors durch Verringerung der Abmessungen der Magneten in Radialrichtung zu verringern.

Beispielsweise kann wenigstens ein Magnet aus keilförmigen Segmenten bestehen, die in Axialrichtung zusammen- und auseinandergeschoben werden können und beim Auseinanderschieben insgesamt in Radialrichtung gesehen weniger Raum beanspruchen als im zusammengeschobenen Zustand. Es kann jedoch auch eine Teilung in verschiedenen anderen Ebenen vorgesehen sein, wobei Verschiebungen der einzelnen Segmente eines Magneten auch in Umfangsrichtung des Rotors und/oder in Radialrichtung des Motors denkbar sind. Auch die Segmente eines Magneten können als Magnetelemente bezeichnet werden, so dass unter dem Begriff Magnetelemente sowohl die Segmente eines Magneten als auch ganze Magneten zusammengefasst werden.

Wichtig ist, dass die beschriebenen Bewegungen der Magneten oder der Segmente von Magneten zu einer Durchmesserverringerung führen, reversibel sind und in einfacher Weise für eine nachfolgende Expansion des Rotors rückgängig gemacht werden können.

Um eine möglichst einfache radiale Komprimierung eines Stators zu ermöglichen, kann beispielsweise vorteilhaft vorgesehen sein, dass die Wicklungsanordnung wenigstens eine Teilwicklung aufweist, die reversibel verformbar ist. Beispielsweise können derartige Teilwicklungen elastisch ausgebildet sein und beispielsweise elastische Leiter enthalten, die eine vorübergehende Verformung einer Teilwicklung zulassen. Eine derartige Verformung kann sowohl elastisch als auch plastisch vorgesehen sein.

Es kann auch vorgesehen sein, dass die Wicklungsanordnung wenigstens zwei Teilwicklungen aufweist, die gegeneinander reversibel verschiebbar sind. Derartige Teilwicklungen können beispielsweise unvergossen oder in einem starren oder elastischen Vergusswerkstoff vergossen sein und bei der radialen Kompression des Stators schindelartig übereinandergeschoben werden, insbesondere in Umfangsrichtung des Stators. Es ist jedoch auch ein Schwenken oder Drehen von Teilwicklungen denkbar, sofern der Rotor aus dem Stator entfernt worden ist.

Zur besseren Verformbarkeit der Wicklungsanordnung kann beispielsweise vorgesehen sein, dass die Wicklungsanordnung wenigstens eine in einem elastischen Werkstoff vergossene Teilwicklung aufweist. Beispielsweise kann eine Teilwicklung in einem Elastomer vergossen sein, zum Beispiel in einem Silikonelastomer oder in einem Kautschukwerkstoff. Es können auch größere Teile der Wicklungsanordnung, beispielsweise auch die gesamte Wicklungsanordnung, in einem derartigen elastischen Werkstoff vergossen sein.

Um den Wirkungsgrad zu erhöhen, kann die elastische Matrix insbesondere auf der Außenseite mit einem ferromagnetischen Füllstoff versehen werden.

Sind einzelne Teile der Wicklungsanordnung jeweils für sich separat vergossen, so kann auch die elastische Verformbarkeit mit einer Verschiebbarkeit kombiniert sein, beispielsweise wenn die Teilwicklungen elastische Leiter aufweisen und/oder in einem elastischen Werkstoff vergossen sind.

Beispielsweise kann auch vorgesehen sein, dass die Wicklungsanordnung wenigstens teilweise aus einer Gedächtnislegierung bestehende Leiter aufweist. In diesem Fall kann eine Teilwicklung oder die gesamte Wicklungsanordnung beispielsweise durch gezieltes Einstellen einer Solltemperatur am Zielort eine gewünschte Form und Größe einnehmen. Bei medizinischen Anwendungen kann die Legierung beispielsweise so eingestellt werden, dass beim Annehmen einer Körpertemperatur eines Patienten die Wicklungsanordnung die gewünschte Sollform annimmt.

Um im Fall einer aus mehreren gegeneinander bewegbaren Teilwicklungen bestehenden Wicklungsanordnung ein wiederholtes Komprimieren und Expandieren und einen reproduzierbaren Ablauf der Kompressions- und Expansionsbewegung zu gewährleisten, kann beispielsweise auch vorteilhaft vorgesehen sein, dass die Wicklungsanordnung zwischen gegeneinander bewegbaren Elementen definierte Biege- und/oder Knickbereiche aufweist. Derartige Biege- und/oder Knickbereiche können in Form von weichen und/oder flexiblen Leiterteilen, beispielsweise durch Vorsehen von als Litze ausgebildeten Längenabschnitten der Leiter oder durch besonders dünn gestaltete Leiterbereiche, realisiert sein.

Um bei einem Motor der oben beschriebenen Art die Verschiebung von Rotor und Stator gegeneinander in der Sollposition auch aus einiger Entfernung bewirken zu können, sieht die Erfindung vorteilhaft ein Verbindungselement vor, das sich vom Motor weg erstreckt und mittels dessen Rotor und Stator in Axialrichtung gegeneinander verschiebbar sind. Das Verbindungselement kann als typisches Manipulationselement, beispielsweise nach Art eines Bowdenzuges oder ähnlich, ausgebildet sein, wobei verschiedene Teile des Verbindungselements mit dem Stator einerseits und dem Rotor andererseits verbunden sein können.

Mittels des Verbindungselements ist eine Relativverschiebung von Stator und Rotor und damit in der Sollposition ein Einziehen des Rotors in den Stator möglich, wobei entweder vorher der Stator bereits expandiert wurde oder durch Einziehen des Rotors in den Stator dieser radial expandiert wird.

Weiter bezieht sich die Erfindung auf eine Implantieranordnung mit einem Hohlkatheter sowie einem in diesem komprimiert angeordneten Stator und einem Rotor. Im Rahmen einer derartigen Implantieranordnung kann der radial komprimierbare Motor in einfacher Form in einen Hohlkatheter eingezogen werden, wobei der Motor üblicherweise im Hohlkatheter in der komprimierten Form aufgenommen ist. Der Hohlkatheter kann dann beispielsweise mittels einer Schleuse in ein Blutgefäß eines Patienten eingebracht, durch dieses verschoben und zu einer Sollposition, beispielsweise in einen Aortenbogen, in eine Herzklappe oder in ein Ventrikel eingebracht werden. Danach kann der Hohlkatheter zurückgezogen werden, wobei der Motor aus diesem herausgeschoben und entweder dabei oder darauf radial expandiert wird.

Die Erfindung bezieht sich außer auf einen Motor der oben beschriebenen Art und auf eine Implantieranordnung auch auf ein Verfahren zum Positionieren eines Motors der beschriebenen Art, wobei der Stator und der Rotor durch einen Kanal zu einer Zielposition verschoben werden, wobei wenigstens der Stator radial komprimiert ist, und dass darauf wenigstens der Stator radial expandiert wird.

Eine vorteilhafte Ausgestaltung des Verfahrens sieht vor, dass nach dem Verschieben von Stator und Rotor zur Zielposition der Stator und der Rotor gegeneinander in Axialrichtung verschoben werden.

Die Erfindung bezieht sich weiterhin auf eine Pumpe, insbesondere Blutpumpe, enthaltend einen Motor mit einem Stator und einem um die Axialrichtung antreibbaren Rotor, wobei von diesen wenigstens einer, insbesondere der Stator, welcher eine strombeaufschlagbare Wicklungsanordnung aufweist, radial komprimierbar und expandierbar ist. Vorteil ist hierbei, dass der Motor und die Pumpe stark ineinander integriert sind. Hierdurch wird es möglich, Pumpen besonders kleinbauend herzustellen; insbesondere ist vorteilhaft, dass Pumpen mit einem sehr geringen radialen Durchmesser zu einem Einsatzort verbracht werden können und dort dann radial expandiert werden können, um die eigentliche Pumpleistung zu leisten.

Eine Ausführungsform sieht hierbei vor, dass der Rotor mit einem Pumprotor verbunden ist, wobei der Pumprotor eine Beschaufelung zur Förderung von Fluiden aufweist. Beispielsweise kann der Pumprotor auf den eigentlichen magnetischen Rotor aufgebracht sein bzw. diesen radial umfassen; es ist allerdings auch möglich, dass diese anderweitig miteinander verbunden sind, beispielsweise dass der magnetische Rotor in den Pumprotor eingegossen/ eingelassen ist.

Eine weitere Ausführungsform sieht vor, dass der Pumprotor sich im Betriebszustand zumindest teilweise innerhalb des Stators befindet. Hierdurch ergibt sich im Betriebszustand eine radiale Schichtung, die von radial außen ausgehend bis zum Mittelpunkt wie folgt ist: 1. Stator, 2. Pumprotor, 3. magnetischer Rotor. In Sonderformen, bei denen der Pumprotor und der magnetische Rotor miteinander verbunden sind, kann dies möglicherweise auch anders sein.

Eine vorteilhafte Ausführungsform sieht vor, dass der Pumprotor radial komprimierbar ist, insbesondere dass der Pumprotor radial elastisch komprimierbar ist. Eine Variante sieht hierbei vor, dass primär die Beschaufelung des Pumprotors elastisch komprimierbar ist und sich beispielsweise an die Nabe des Pumprotors anlegt.

Es sind verschiedene Varianten der erfindungsgemäßen Pumpe möglich, wobei auch alle Varianten der Motoren nach der vorliegenden Erfindung für die Pumpe nutzbar sind.

Im Folgenden wird die Erfindung anhand von Ausführungsbeispielen in verschiedenen Figuren gezeigt und nachfolgend beschrieben. Dabei zeigt
- Fig. 1: einen Motor mit einem Rotor und einem Stator in einer schematischen Darstellung in einem Längsschnitt,
- Fig. 2: den Motor aus Figur 1 in einer komprimierten Form, wobei Stator und Rotor axial auseinandergezogen sind,
- Fig. 3: schematisch in einer abgewickelten Form Einzeldrähte der Wicklungsanordnung mit einer Längenreserve in Form eines spiraligen bzw. mäanderförmigen Verlaufs,
- Fig. 4a: eine Darstellung einer Wicklungsanordnung in Axialsicht gesehen, im linken Teil der Figur in spiraliger Form des Leiters komprimiert, rechts expandiert,
- Fig. 4b: eine Wicklungsanordnung in Axialrichtung gesehen, im linken Teil der Figur in Mäanderform des Leiters komprimiert, im rechten Teil radial expandiert,
- Fig. 5: in einer dreidimensionalen Ansicht eine Wicklungsanordnung mit einer Mehrzahl von Teilwicklungen, die im abgerollten Zustand im Wesentlichen eine rauten- oder trapezförmige Ausbildung aufweisen,
- Fig. 6: schematisch in einer dreidimensionalen Ansicht den Verlauf einer einzelnen Windung einer Teilwicklung aus der Figur 5,
- Fig. 7: in einer Ansicht in Axialrichtung schematisch die Anordnung verschiedener Teilwicklungen zueinander am Umfang der Wicklungsanordnung im radial expandierten Zustand,
- Fig. 8: die Anordnung von Teilwicklungen aus Figur 7 im radial komprimierten Zustand,
- Fig. 9: schematisch einen Längsschnitt durch einen Motor mit einem Stator und einem Rotor, wobei der Rotor gekapselt ist, in zusammengesetztem, expandiertem Zustand,
- Fig. 10: den Motor aus Figur 9 im komprimierten, axial auseinandergezogenen Zustand,
- Fig. 11: einen Stator und einen Rotor, die axial hintereinander angeordnet sind, wobei der Stator radial komprimiert ist und beide eine Manipulationseinrichtung aufweisen,
- Fig. 12: einen Querschnitt durch einen Stator, dessen Wicklungsanordnung in Umfangsrichtung in vier Teilwicklungen geteilt ist,
- Fig. 13: einen komprimierten Zustand des Stators aus Figur 12, bei dem die Teilwicklungen durch radialen Druck mechanisch radial nach innen umgeschlagen sind,
- Fig. 14: eine Darstellung des Stators aus den Figuren 12 und 13 in weiter komprimiertem Zustand,
- Fig. 15: in Axialrichtung schematisch die Darstellung eines Rotors mit zwei Magneten,
- Fig. 16: eine Seitenansicht eines in Segmente geteilten und radial komprimierbaren Magneten eines Rotors,
- Fig. 17: eine Darstellung eines Stators mit einer in Axialrichtung teilbaren Wicklungsanordnung,
- Fig. 18: eine Darstellung eines Stators mit einem darin angeordneten Rotor sowie einem auf dem Rotor angeordneten komprimierbaren Pumprotor im expandierten Zustand,
- Fig. 19: eine Darstellung der Vorrichtung aus Fig. 18 im komprimierten Zustand sowie
- Fig. 20: eine Darstellung eines Stators mit einem axial aus diesem herausgezogenen Pumprotor in einer Seitenansicht.

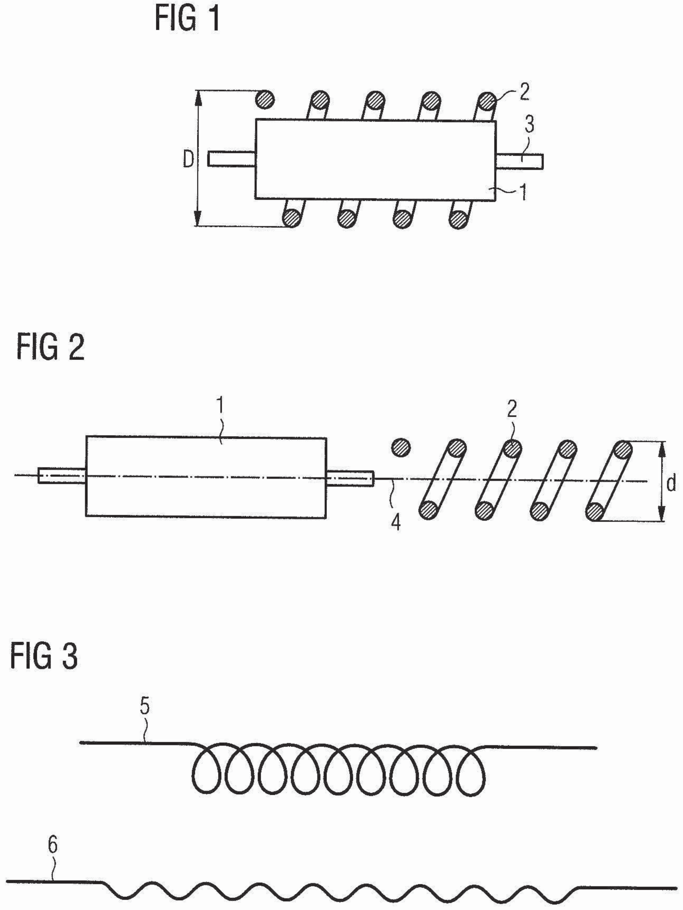

In Figur 1 ist schematisch in einem Längsschnitt ein Stator 2 und ein Rotor 1 eines Elektromotors dargestellt. Weitere Teile sowie Details sind der Übersichtlichkeit halber weggelassen. Der Stator weist eine schematisch angedeutete zylindrische Wicklungsanordnung auf, die aus einer oder mehreren Teilwicklungen bestehen kann. Der Rotor 1 weist wenigstens einen Permanentmagneten sowie eine Nabe auf, die mit einer Welle 3 verbunden ist. Die magnetischen Pole des Rotors 1 bzw. seines/seiner Magneten sind im Magnetfeld des Stators 2 antreibbar. Die Welle 3 ist üblicherweise an einer oder mehreren Stellen in Gleit- oder Kugellagern drehbar gelagert. Die Lager können beispielsweise mit dem Stator 2 oder mit einem nicht dargestellten Gehäuse des Stators bzw. des gesamten Motors fest verbunden sein. In Figur 1 ist ersichtlich, dass der Stator, der den Rotor 1 konzentrisch und koaxial umgibt, in dem dort dargestellten radial expandierten Zustand einen Durchmesser D aufweist.

Figur 2 zeigt die bereits in Figur 1 dargestellten Elemente eines Motors, nämlich einen Rotor 1 sowie eine Wicklungsanordnung eines Stators 2, wobei Rotor und Stator in Axialrichtung 4 auseinandergezogen sind. Der Stator und der Rotor überlappen einander in Axialrichtung in diesem Zustand nicht. Der Stator ist durch radiale Kompression der Wicklungsanordnung insgesamt radial auf den Durchmesser d komprimiert, der gleich oder kleiner als der Außendurchmesser des Rotors 1 ist.

Es wird somit deutlich, dass durch die Teilbarkeit des Motors und die Verschiebbarkeit des Stators gegenüber dem Rotor der Stator, sobald der Rotor aus ihm entfernt wurde, radial komprimierbar ist.

Unabhängig davon und zusätzlich kann auch der Rotor in Radialrichtung komprimierbar sein. In diesem Fall können Stator und Rotor auch im zusammengebauten Zustand gemeinsam radial komprimiert werden, oder sie können axial gegeneinander verschoben und beide getrennt voneinander radial komprimiert werden. Im letztgenannten Fall ist es sinnvoll, aber nicht notwendig, dass beide Elemente, d. h. sowohl der Stator als auch der Rotor, etwa auf den gleichen Außendurchmesser komprimierbar sind.

Figur 3 zeigt im oberen Bereich einen ersten Leiter 5 einer Wicklungsanordnung eines erfindungsgemäßen Motors im komprimierten Zustand, wobei der Leiter spiralförmig verläuft. Wird aus diesem spiralförmig verlaufenden Leiter eine Wicklungsanordnung oder Teilwicklung gebildet, so lässt sich diese unter Streckung des Wicklungsdrahtes 5 radial expandieren und später radial wieder komprimieren. Im unteren Bereich der Figur 3 ist ein Leiter 6 dargestellt, der im komprimierten Zustand eine Mäanderform aufweist, die beim Übergang in einen expandierten Zustand gestreckt werden kann.

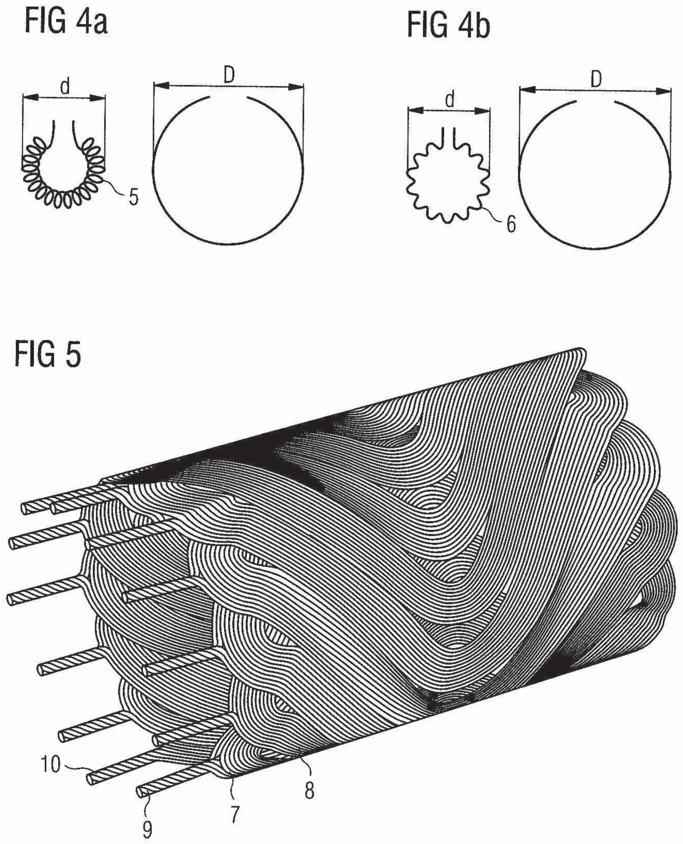

In Figur 4a ist schematisch im linken Teil ein im komprimierten Zustand spiralförmiger Leiter 5 dargestellt, der ebenfalls im komprimierten Zustand schematisch in Form eines Kreisrings dargestellt ist, der eine Wicklungsanordnung symbolisiert. Im rechten Teil der Figur 4a ist in axialer Ansicht eine expandierte Form des Stators dargestellt, bei der der/die Wicklungsleiter gestreckt sind und entsprechend die Wicklungsanordnung und/oder die Teilwicklungen ebenfalls expandiert sind. Der Stator weist im rechten Teil der Figur 4a den vergrößerten Durchmesser D auf, während er im komprimierten, in der linken Hälfte der Figur 4a dargestellten Zustand den verkleinerten Durchmesser d aufweist.

In Figur 4b ist ein komprimierter Leiter 6 in Mäanderform dargestellt, der, in Axialrichtung gesehen, in eine Kreisringform gelegt ist, die eine Wicklungsanordnung eines Stators repräsentiert. Die Anordnung weist den komprimierten Außendurchmesser d auf. Im rechten Teil der Figur 4b ist derselbe Stator im radial expandierten Zustand dargestellt, wobei der/die Wicklungsleiter gestreckt sind, oder zumindest weiter gestreckt als im komprimierten Zustand.

Der Übergang zwischen dem komprimierten und expandierten Zustand des Stators kann beispielsweise durch eine Krafteinwirkung geschehen, indem der Stator durch radialen Außendruck in eine komprimierte Form gebracht wird und bei Wegfallen der äußeren radialen Kompressionskraft elastisch von selbst wieder expandiert.

Es kann auch umgekehrt vorgesehen sein, dass der Stator ohne äußere Krafteinwirkung einen verringerten Durchmesser aufweist und durch Krafteinwirkung expandierbar ist.

Als weitere Alternative kann vorgesehen sein, dass die Wicklungsanordnung Leiter aus sogenannten Gedächtnislegierungen aufweist, die beispielsweise bei Temperaturwechseln ihre Form ändern und in definierten Temperaturbereichen jeweils reproduzierbare Formen aufweisen. Solche Gedächtnislegierungen können beispielsweise NiTi (Nickel-Titan; Nitinol), NiTiCu (Nickel-Titan-Kupfer), CuZn (Kupfer-Zink), CuZnAl (Kupfer-Zink-Aluminium), CuAINi (Kupfer-Aluminium-Nickel), FeNiAl (Eisen-Nickel-Aluminium) oder FeMnSi (Eisen-Mangan-Silizium) sein. Derartige Legierungen werden auch als hyperelastische Legierungen bezeichnet.

Zusätzlich zu den beschriebenen Eigenschaften der Wicklungsanordnung kann auch ein Verguss der gesamten Wicklungsanordnung oder einzelner Teilwicklungen in einen elastischen Werkstoff, wie beispielsweise ein Silikonelastomer oder einen Kautschuk, vorgesehen sein, der für sich elastisch verformbar ist. Es kann auch gar kein Verguss der Wicklungsanordnung oder ein Verguss in einen unelastischen Werkstoff vorgesehen sein, wobei der Verguss einzelner Teilwicklungen separat erfolgt und die Teilwicklungen mitsamt dem jeweiligen Vergusswerkstoff gegeneinander beweglich sind. Auf derartige Konfigurationen wird weiter unten noch näher eingegangen.

Figur 5 zeigt in einer perspektivischen Ansicht eine im Wesentlichen hohlzylindrisch ausgebildete Wicklungsanordnung, die aus einer Mehrzahl von Teilwicklungen besteht. Jede Teilwicklung für sich besteht aus mehreren Windungen eines Leiters und weist zwei elektrische Anschlüsse zur Spannungsversorgung und Stromzuführung auf. Es kann auch die Wicklungsanordnung als ganze Anschlussleiter oder elektrische Anschlüsse aufweisen.

Jede Teilwicklung der dargestellten Wicklungsanordnung weist im abgerollten Zustand eine rautenförmige Grundform auf. Die einzelnen Teilwicklungen überlappen einander in Umfangsrichtung der Wicklungsanordnung. Die einzelnen Teilwicklungen 7, 8 der Wicklungsanordnung aus Figur 5 weisen elektrische Anschlüsse 9, 10 zur Strombeaufschlagung der Statorwicklungsanordnung auf.

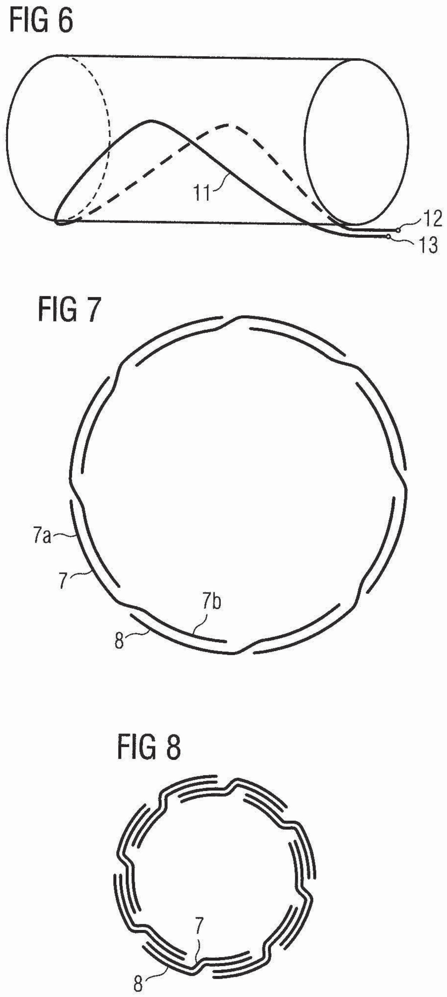

In Figur 6 ist eine einzelne Teilwicklung 7, symbolisiert durch eine einzelne Windung eines Wicklungsleiters, dargestellt und mit dem Bezugszeichen 11 bezeichnet. Die Teilwicklung 11 weist zwei elektrische Anschlüsse 12, 13 zur Strombeaufschlagung auf. In Figur 6 ist schematisch ein Hohlzylinder dargestellt, an dessen Umfang die teilzylindrischen Teilwicklungen, einander überlappend, in Umfangsrichtung gegeneinander versetzt verteilt sind.

In Figur 7 sind in einer Ansicht in Axialrichtung mehrere Teilwicklungen 7, 8 einer Wicklungsanordnung schematisch gezeigt. Die einzelnen Teilwicklungen 7, 8 weisen jeweils einen radial außenliegenden Teil 7a und einen radial innenliegenden Teil 7b auf, wobei der jeweils radial innenliegende Teil von der folgenden Teilwicklung 8, und zwar von deren radial außenliegendem Teil, überdeckt ist. Auf diese Weise ergibt sich eine dachziegelartige Verschachtelung der Teilwicklungen entlang der Umfangslinie des Stators.

Sind die Teilwicklungen gegeneinander beweglich, so können diese schindelartig weiter übereinandergeschoben werden, und damit kann der Durchmesser der Gesamtanordnung sowie der Umfang der Wicklungsanordnung verringert werden. Ein Beispiel eines komprimierten Zustands einer solchen Kompressionsbewegung ist in Figur 8 gezeigt, in der jeweils zwei Teilwicklungen 7, 8 derart übereinandergeschoben sind, dass sie einander vollständig in Umfangsrichtung der Wicklungsanordnung überlappen. Diese Übereinanderschiebbarkeit der einzelnen Teilwicklungen ist mit unvergossenen Teilwicklungen ebenso wie mit vergossenen Teilwicklungen denkbar. Sind die einzelnen Teilwicklungen für sich vergossen, so ist es vorteilhaft, wenn der Vergießwerkstoff leichtes Gleiten zweiter aus ihm bestehender Körper gegeneinander ermöglicht.

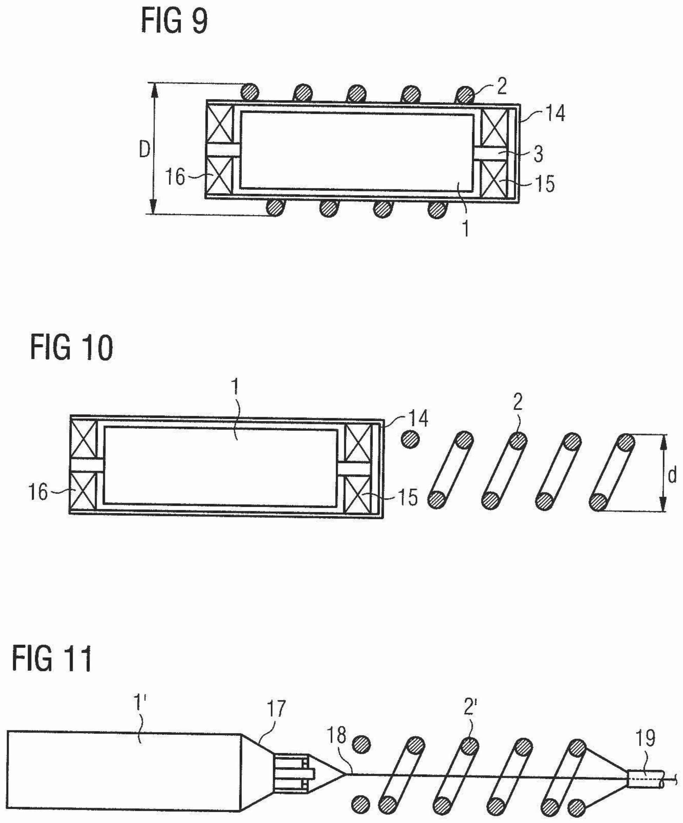

Figur 9 zeigt in einem Längsschnitt einen Motor mit einem radial expandierten Stator 2 sowie einem Rotor 1, der eine Kapselung 14 in Form eines Hohlzylinders aufweist, der den Magnetkörper des Rotors umgibt und der beispielsweise auch die Lager 15, 16 trägt. In den Lagern 15, 16, die als Gleit- oder Kugellager ausgebildet sein können, ist die Welle 3 des Rotors reibungsarm gelagert. Der Durchmesser des Gesamtaufbaus des Motors gemäß Figur 9 im expandierten, zusammengebauten und betriebsbereiten Zustand ist mit D angegeben.

Im Gegensatz dazu ist in Figur 10 derselbe Motor mit denselben Elementen, also einem innerhalb einer Kapselung 14 gekapselten Rotor und einem Stator 2 mit einer Wicklungsanordnung im komprimiertem Zustand dargestellt, wobei der Stator 2 gegenüber dem Rotor 1 in Axialrichtung so weit verschoben ist, dass der Rotor sich außerhalb des Stators befindet. Der Stator 2 ist dann unabhängig vom Rotor 1 radial bis auf den Außendurchmesser des Rotors komprimierbar.

Figur 11 zeigt eine Gestaltung eines Motors mit einem Rotor 1' und einem Stator 2', wobei diese in komprimierter, axial auseinandergezogener Stellung dargestellt sind. Der Rotor 1' weist eine Kapselung auf, in der sich die Magnetanordnung des Rotors, gestützt durch zwei Lager, drehen kann. Die Kapselung des Rotors weist eine konische Zuspitzung 17 sowie eine Verbindung zu einem strangförmigen Manipulationselement 18 auf, das an der Kapselung oder an einem Lager befestigt ist und eine axiale Relativbewegung des Rotors gegenüber dem Stator 2' ermöglicht. Gleichzeitig ist der Stator 2' mit einem zweiten Manipulationselement 19, beispielsweise in Form eines Rohres oder Schlauches, verbunden, durch den hindurch beispielsweise das Manipulationselement 18 geführt sein kann. Von einem entfernten Ort aus können die Manipulationselemente 18, 19, die gemeinsam ein Verbindungselement zu dem Motor bilden, gemeinsam betätigt werden, um eine Relativbewegung von Stator und Rotor gegeneinander auszuführen und beispielsweise durch das Einführen der Kapselung der Rotors 1' in die Wicklungsanordnung des Stators 2' diesen radial aufzuweiten.

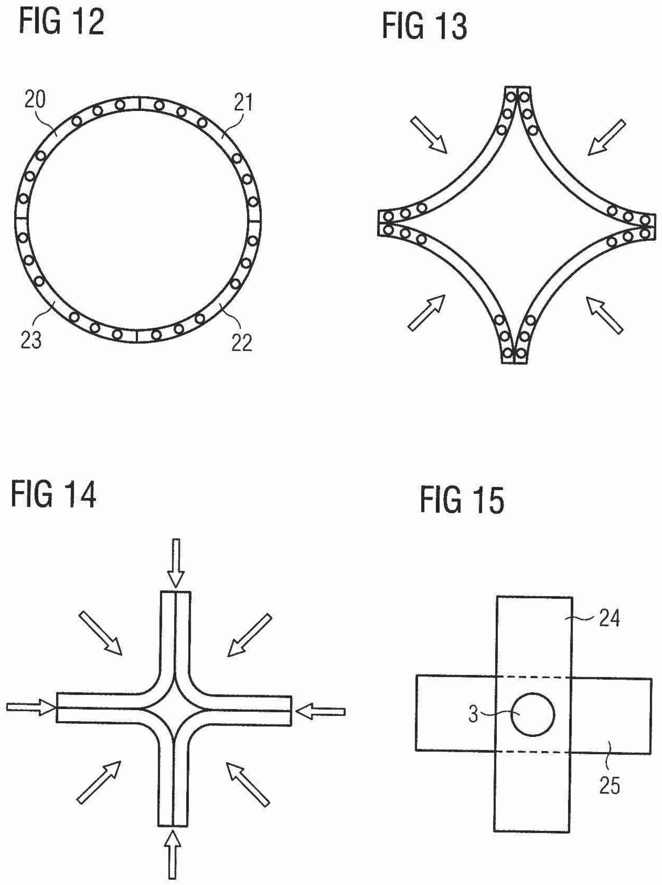

Figur 12 zeigt eine besondere Wicklungsanordnung, bestehend aus vier separaten, jeweils separat in einem elastischen Werkstoff vergossenen Teilwicklungen 20, 21, 22, 23. Jede dieser Teilwicklungen weist eine teilhohlzylindrische Form auf, und die Teilwicklungen lassen sich mit ihren Vergusskörpern zu einem gesamten Hohlzylinder zusammensetzen.

Wird radial von außen eine Kraft auf die Wicklungsanordnung ausgeübt, so ergibt sich die Konstellation, wie sie in Figur 13 dargestellt ist, wobei die einzelnen Vergusskörper und Teilwicklungen radial nach innen umschlagen. Die einzelnen Vergusskörper der Teilwicklungen können beispielsweise durch Filmgelenke miteinander beweglich verbunden sein. In dem in Figur 13 dargestellten Zustand nimmt die Wicklungsanordnung bereits in radialer Richtung einen wesentlich geringeren Raum ein als in der in Figur 12 dargestellten Form. Bei einer weiteren radialen Kompression werden die einzelnen Teilwicklungen weiter radial nach innen komprimiert, was durch eine Verformbarkeit der Vergusskörper zusätzlich ermöglicht ist. Es ergibt sich bei voller Kompression die in Figur 14 dargestellte Gestalt. Diese kann bei Wegfall der radial nach innen wirkenden Kompressionskräfte selbsttätig wieder in die in Figur 12 dargestellte Form expandierbar sein, wobei die Rückstellkräfte beispielsweise durch die elastisch verformten Vergusskörper, jedoch auch durch die Wicklungsleiter selbst oder durch beide gemeinsam aufgebracht werden können. Werden die einzelnen Teilwicklungen nicht vergossen, so kann auch innerhalb jeder Teilwicklung eine entsprechende Verformung der Wicklungsleiter reversibel stattfinden.

Figur 15 zeigt in einer Darstellung in Axialrichtung zwei senkrecht aufeinanderstehende Magneten 24, 25, die im magnetischen Feld der Wicklungsanordnung antreibbar sind. Die Magneten 24, 25 sind mit der Welle 3 des Rotors fest verbunden.

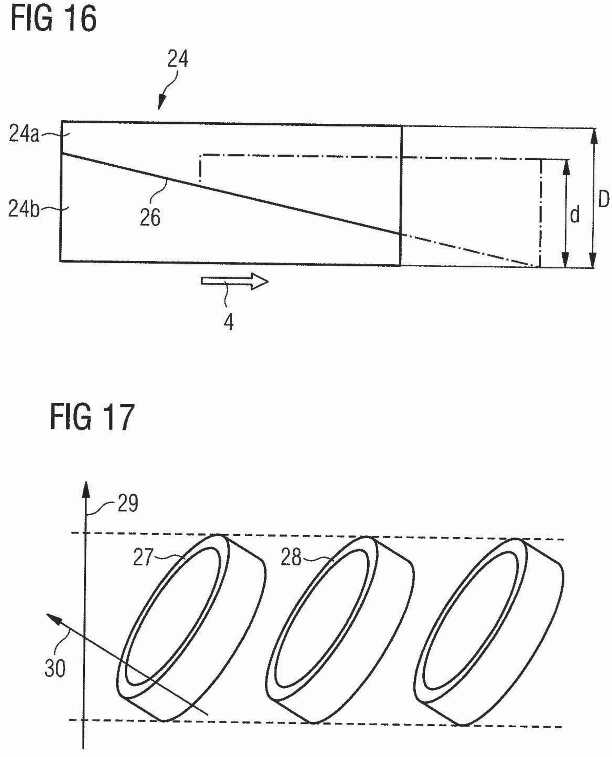

In Figur 16 ist die Teilung eines Magneten 24 entlang der Fläche 26 dargestellt, wodurch zwei Segmente 24a, 24b des Magneten 24 entstehen, die jeweils ein Magnetelement bilden. Der Magnet 24 weist in dem durchgezogen dargestellten Zustand die Form eines Quaders auf. In gestrichelter Darstellung ist die Konstellation gezeigt, in der das Segment 24a entlang der Fläche 26 gegenüber dem Segment 24b in Axialrichtung 4 verschoben ist. Es ergibt sich in Axialrichtung eine Verlängerung des Magneten 24 und in einer radialen Richtung eine Kompression vom Durchmesser D auf den Durchmesser d, wie in Figur 16 im rechten Teil angegeben ist. Durch die gezeigte Konstruktion des Rotors kann auch dieser radial komprimierbar gestaltet werden, so dass der Motor entweder im zusammengebauten Zustand durch gemeinsame Kompression von Stator und Rotor oder im axial auseinandergezogenen Zustand auch allein durch radiale Kompression des Stators komprimierbar ist.

Der Motor kann damit zum Verbringen an seinen Einsatzort komprimiert werden; beispielsweise kann er als Antriebsaggregat für eine Blutpumpe implantierbar gestaltet sein und durch ein Blutgefäß in komprimiertem Zustand innerhalb eines Patientenkörpers zu einem Einsatzort verschoben werden. Dort kann der Motor expandiert werden, ebenso wie beispielsweise eine Blutpumpe, und der Motor kann im expandierten Zustand die notwendigen Drehmomente bzw. die benötigte Leistung zum Antrieb einer Pumpe aufbauen.

Figur 17 zeigt eine Gestaltungsvariante, bei der die Wicklungsanordnung in mehrere Teilwicklungen 27, 28 aufgeteilt ist, die jeweils kreisringförmig aufgebaut sind und axial hintereinandergeschaltet eine hohlzylindrische Wicklungsanordnung bilden. Werden bei einer solchen Anordnung die kreisringförmigen Teilwicklungen gekippt, so wird der Querschnitt der Wicklungseinrichtung elliptisch, jedoch im Durchmesser gegenüber der nicht gekippten Anordnung, in einer Achse 29 komprimiert. In der hierauf senkrecht stehenden Achse 30 bleibt der Durchmesser gleich. Dennoch ergibt sich beim Kippen gegebenenfalls eine zur Positionierung des Motors günstigere Form. Die Kippung kann jederzeit nach der Positionierung des Motors rückgängig gemacht werden.

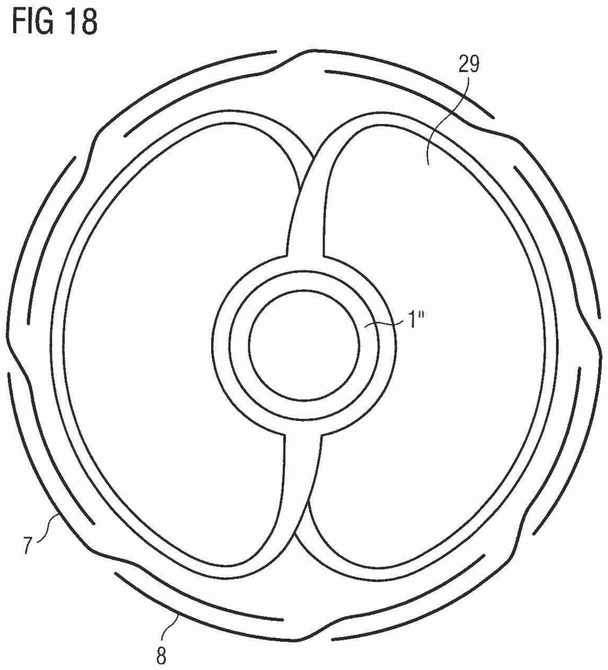

Fig. 18 zeigt eine Vorrichtung aus einem Stator gemäß Fig. 7 mit den dort bereits beschriebenen Wicklungen 7, 8. In diesem Stator befindet sich ein magnetischer Rotor 1", mit dem ein radial komprimierbarer Pumprotor 29 derart verbunden ist, dass er gemeinsam mit dem Rotor 1" um die gleiche Achse rotieren kann. Im Ausführungsbeispiel ist der Pumprotor aus einem elastischen, vorzugsweise hyperelastischen Kunststoff ausgeführt, der es ermöglicht, dass sich der Pumprotor 29 beim Komprimieren des Stators zusammenfaltet und beim Expandieren des Stators wieder elastisch bzw. hyperelastisch in die Ausgangsform zurück expandiert.

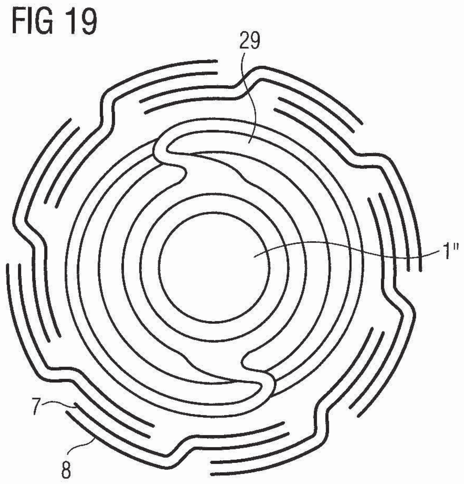

In Fig. 19. ist schematisch dargestellt, wie sich der Stator analog Fig. 8 komprimiert, wobei der Pumprotor 29 ebenfalls eine komprimierte Form annimmt. Hierbei falten sich die Schaufeln des Pumprotors um die Achse des Rotors herum und legen sich an die Nabe des Pumprotors an.

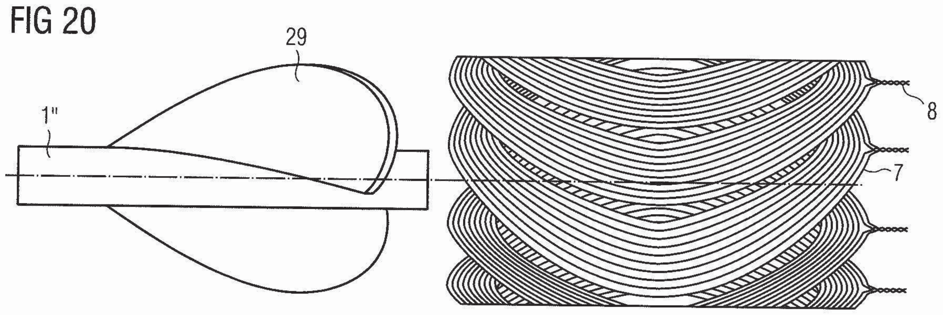

Prinzipiell kann die Vorrichtung auch so ausgeführt werden, dass der Rotor 1" gemeinsam mit dem Pumprotor 29 axial entsprechend Fig. 20 aus dem Stator herausgezogen werden kann. Dadurch werden der Pumprotor 29 und der Stator axial hintereinander angeordnet. In diesem Zustand können der Stator und der Pumprotor gemeinsam komprimiert werden, was gegenüber der Ausführung in Fig. 19 eine weitere Reduzierung des komprimierten Durchmessers ermöglicht.

Der Pumprotor kann prinzipiell auf sehr verschiedene Weise ausgeführt werden. Neben der in den Figuren 18 und 19 ausgeführten Variante aus elastischem bzw. hyperelastischem Kunststoff sind verschiedene andere Varianten aus dem Stand der Technik, beispielsweise aus US 4,753,221; US 5,749,855; US 7,393,181; US 2009/0062597 A1; EP 2047873 A1;
US 2011 / 0275884 A1; EP 2229965 A1; WO 2010 149393 A1; EP 2299119 A1; EP 2338540 A1; EP 2338541 A1; EP 2363157; EP 2407185 A1; EP 2407187 A1; EP 2407186 A1 bekannt.

Die Anmeldung beinhaltet, unter anderem, die folgenden Aspekte:
1. Motor mit einem Stator (2, 2') und einem um eine Axialrichtung (4) antreibbaren Rotor (1, 1'), dadurch gekennzeichnet, dass von diesen wenigstens einer, insbesondere der Stator, welcher eine strombeaufschlagbare Wicklungsanordnung (7, 8, 20, 21, 22, 23, 27, 28) aufweist, radial komprimierbar und expandierbar ist.
2. Motor nach Aspekt 1, dadurch gekennzeichnet, dass Rotor (1, 1') und Stator (2, 2') gegeneinander in Axialrichtung (4) verschiebbar sind zwischen einer ersten Position, in der der Stator (2, 2') radial komprimierbar ist, und einer zweiten Position, in der der Stator radial expandiert ist.
3. Motor nach Aspekt 1 oder 2, dadurch gekennzeichnet, dass der Rotor (1, 1') in Radialrichtung komprimierbar ist.
4. Motor nach Aspekt 3, dadurch gekennzeichnet, dass der Rotor (1, 1') eine Mehrzahl von Magnetelementen (24, 24a, 24b, 25) aufweist, die insbesondere in Axialrichtung (4) gegeneinander reversibel bewegbar sind.
5. Motor nach Aspekt 1 oder einem der folgenden, dadurch gekennzeichnet, dass die Wicklungsanordnung (7, 8, 20, 21, 22, 23, 27, 28) wenigstens eine Teilwicklung (7, 8, 20, 21, 22, 23, 27, 28) aufweist, die reversibel verformbar ist.
6. Motor nach Aspekt 1 oder einem der folgenden, dadurch gekennzeichnet, dass die Wicklungsanordnung (7, 8, 20, 21, 22, 23, 27, 28) wenigstens zwei Teilwicklungen (7, 8, 20, 21, 22, 23, 27, 28) aufweist, die gegeneinander reversibel verschiebbar sind.
7. Motor nach Aspekt 6, dadurch gekennzeichnet, dass die Teilwicklungen schindelartig übereinanderschiebbar sind.
8. Motor nach Aspekt 1 oder einem der folgenden, dadurch gekennzeichnet, dass die Wicklungsanordnung (7, 8, 20, 21, 22, 23, 27, 28) wenigstens eine in einem elastischen Werkstoff vergossene Teilwicklung aufweist.
9. Motor nach Aspekt 1 oder einem der folgenden, dadurch gekennzeichnet, dass verschiedene Teilwicklungen (7, 8, 20, 21, 22, 23, 27, 28) der Wicklungsanordnung in separaten, gegeneinander beweglichen Teilkörpern vergossen sind.
10. Motor nach Aspekt 1 oder einem der folgenden, dadurch gekennzeichnet, dass die Wicklungsanordnung (7, 8, 20, 21, 22, 23, 27, 28) wenigstens teilweise aus einer Gedächtnislegierung bestehende Leiter (5, 6) aufweist.
11. Motor nach Aspekt 1 oder einem der folgenden, dadurch gekennzeichnet, dass die Wicklungsanordnung (7, 8, 20, 21, 22, 23, 27, 28) zwischen gegeneinander bewegbaren Elementen definierte Biege- und/oder Knickbereiche aufweist.
12. Motor nach Aspekt 1 oder einem der folgenden, gekennzeichnet durch ein Verbindungselement (18, 19), das sich vom Motor weg erstreckt und mittels dessen Rotor (1, 1') und Stator (2, 2') in Axialrichtung (4) gegeneinander verschiebbar sind.
13. Implantieranordnung mit einem Hohlkatheter sowie einem in diesem komprimiert angeordneten Motor gemäß Aspekt 1 oder einem der folgenden Ansprüche.
14. Verfahren zum Positionieren eines Motors nach einem der Aspekte 1 bis 12, dadurch gekennzeichnet, dass der Stator (2, 2') und der Rotor (1, 1') durch einen Kanal zu einer Zielposition verschoben werden, wobei wenigstens der Stator radial komprimiert ist, und dass darauf wenigstens der Stator radial expandiert wird.
15. Verfahren nach Aspekt 14, dadurch gekennzeichnet, dass nach dem Verschieben von Stator (2, 2') und Rotor (1, 1') zur Zielposition der Stator und der Rotor gegeneinander in Axialrichtung (4) verschoben werden.
16. Pumpe, insbesondere Blutpumpe, enthaltend einen Motor mit einem Stator (2, 2') und einem um eine Axialrichtung (4) antreibbaren Rotor (1, 1'), wobei von diesen wenigstens einer, insbesondere der Stator, welcher eine strombeaufschlagbare Wicklungsanordnung (7, 8, 20, 21, 22, 23, 27, 28) aufweist, radial komprimierbar und expandierbar ist.
17. Pumpe nach Aspekt 16, dadurch gekennzeichnet, dass der Rotor mit einem Pumprotor verbunden ist, wobei der Pumprotor eine Beschaufelung zur Förderung von Fluiden aufweist.
18. Pumpe nach Aspekt 17, dadurch gekennzeichnet, dass der Pumprotor sich im Betriebszustand zumindest teilweise innerhalb des Stators befindet.
19. Pumpe nach Aspekt 17 oder 18, dadurch gekennzeichnet, dass der Pumprotor radial komprimierbar ist.
20. Pumpe nach Aspekt 17, 18 oder 19, dadurch gekennzeichnet, dass der Pumprotor radial elastisch komprimierbar ist.
21. Pumpe mit einem komprimierbaren Pumprotor, wobei der Pumprotor in einem Motor gemäß Aspekt 1 bis 12 angeordnet ist.

## Patentansprüche

1. Motor mit einem Stator (2, 2') und einem um eine Axialrichtung (4) antreibbaren Rotor (1, 1'), **dadurch gekennzeichnet, dass** von diesen wenigstens einer, insbesondere der Stator, welcher eine strombeaufschlagbare Wicklungsanordnung (7, 8, 20, 21, 22, 23, 27, 28) aufweist, radial komprimierbar und expandierbar ist.

2. Motor nach Anspruch 1, **dadurch gekennzeichnet, dass** Rotor (1, 1') und Stator (2, 2') gegeneinander in Axialrichtung (4) verschiebbar sind zwischen einer ersten Position, in der der Stator (2, 2') radial komprimierbar ist, und einer zweiten Position, in der der Stator radial expandiert ist.

3. Motor nach Anspruch 1 oder 2, **dadurch gekennzeichnet, dass** der Rotor (1, 1') in Radialrichtung komprimierbar ist und oder dadurch dass der Rotor (1, 1') eine Mehrzahl von Magnetelementen (24, 24a, 24b, 25) aufweist, die insbesondere in Axialrichtung (4) gegeneinander reversibel bewegbar sind.

4. Motor nach Anspruch 1 oder einem der folgenden, **dadurch gekennzeichnet, dass** die Wicklungsanordnung (7, 8, 20, 21, 22, 23, 27, 28) wenigstens eine Teilwicklung (7, 8, 20, 21, 22, 23, 27, 28) aufweist, die reversibel verformbar ist.

5. Motor nach Anspruch 1 oder einem der folgenden, **dadurch gekennzeichnet, dass** die Wicklungsanordnung (7, 8, 20, 21, 22, 23, 27, 28) wenigstens zwei Teilwicklungen (7, 8, 20, 21, 22, 23, 27, 28) aufweist, die gegeneinander reversibel verschiebbar sind, wobei die Teilwicklungen vorzugsweise schindelartig übereinanderschiebbar sind.

6. Motor nach Anspruch 1 oder einem der folgenden, **dadurch gekennzeichnet, dass**
die Wicklungsanordnung (7, 8, 20, 21, 22, 23, 27, 28) wenigstens eine in einem elastischen Werkstoff vergossene Teilwicklung aufweist, und/oder
verschiedene Teilwicklungen (7, 8, 20, 21, 22, 23, 27, 28) der Wicklungsanordnung in separaten, gegeneinander beweglichen Teilkörpern vergossen sind.

7. Motor nach Anspruch 1 oder einem der folgenden, **dadurch gekennzeichnet, dass**
die Wicklungsanordnung (7, 8, 20, 21, 22, 23, 27, 28) wenigstens teilweise aus einer Gedächtnislegierung bestehende Leiter (5, 6) aufweist, und/oder
die Wicklungsanordnung (7, 8, 20, 21, 22, 23, 27, 28) zwischen gegeneinander bewegbaren Elementen definierte Biege- und/oder Knickbereiche aufweist.

8. Motor nach Anspruch 1 oder einem der folgenden, **gekennzeichnet durch** ein Verbindungselement (18, 19), das sich vom Motor weg erstreckt und mittels dessen Rotor (1, 1') und Stator (2, 2') in Axialrichtung (4) gegeneinander verschiebbar sind.

9. Implantieranordnung mit einem Hohlkatheter sowie einem in diesem komprimiert angeordneten Motor gemäß Anspruch 1 oder einem der folgenden Ansprüche.

10. Verfahren zum Positionieren eines Motors nach einem der Ansprüche 1 bis 12, **dadurch gekennzeichnet, dass** der Stator (2, 2') und der Rotor (1, 1') durch einen Kanal zu einer Zielposition verschoben werden, wobei wenigstens der Stator radial komprimiert ist, und dass darauf wenigstens der Stator radial expandiert wird.

11. Verfahren nach Anspruch 10, **dadurch gekennzeichnet, dass** nach dem Verschieben von Stator (2, 2') und Rotor (1, 1') zur Zielposition der Stator und der Rotor gegeneinander in Axialrichtung (4) verschoben werden.

12. Pumpe, insbesondere Blutpumpe, enthaltend einen Motor, insbesondere einen Motor gemäß einem der Ansprüche 1 bis 8, mit einem Stator (2, 2') und einem um eine Axialrichtung (4) antreibbaren Rotor (1, 1'), wobei von diesen wenigstens einer, insbesondere der Stator, welcher eine strombeaufschlagbare Wicklungsanordnung (7, 8, 20, 21, 22, 23, 27, 28) aufweist, radial komprimierbar und expandierbar ist.

13. Pumpe nach Anspruch 12, **dadurch gekennzeichnet, dass** der Rotor mit einem Pumprotor verbunden ist, wobei der Pumprotor eine Beschaufelung zur Förderung von Fluiden aufweist, wobei sich der Pumprotor vorzugsweise im Betriebszustand zumindest teilweise innerhalb des Stators befindet.

14. Pumpe nach Anspruch 13, **dadurch gekennzeichnet, dass** der Pumprotor radial komprimierbar, insbesondere radial elastisch komprimierbar, ist.

15. Pumpe mit einem komprimierbaren Pumprotor, wobei der Pumprotor in einem Motor gemäß Anspruch 1 bis 8 angeordnet ist.
